# EUROPEAN PATENT SPECIFICATION

(11) **EP 2 719 960 B1**
(45) Date of publication and mention of the grant of the patent: **15.07.2020**
(21) Application number: 13164662.2
(22) Date of filing: 22.04.2013
(51) Int. Cl.: F24F 1/00, F24F 11/30, A61L 9/12, A61L 9/22, F24F 1/0007, F24F 3/16, F24F 1/008

(54) **Air conditioner**
Klimaanlage
Climatiseur

(30) Priority: 10.10.2012 KR 20120112296
(43) Date of publication of application: 16.04.2014
(73) Proprietor: LG Electronics, Inc., Yeongdeungpo-gu 150-721 Seoul (KR)
(72) Inventor: Kim, Hyunjung, 641-711 Changwon-si (KR); Jeon, Jongsun, 641-711 Changwon-si (KR); Choi, Jaeseung, 641-711 Changwon-si (KR); Choi, Jieun, 641-711 Changwon-si (KR); Kim, Hayoung, 641-711 Changwon-si (KR); Kim, Woojin, 641-711 Changwon-si (KR); Park, Hyungho, 641-711 Changwon-si (KR)
(74) Representative: Vossius & Partner Patentanwälte Rechtsanwälte mbB

(56) References cited:
- EP-A1- 2 143 575
- EP-A1- 2 143 576
- EP-A1- 2 143 577
- EP-A2- 1 686 326
- WO-A1-2015/163691
- FR-A1- 2 833 887
- FR-A1- 2 867 424
- FR-A1- 2 946 256
- US-A- 5 957 771

## Description

### BACKGROUND

The present disclosure relate to an air conditioner.

Air conditioners are home appliances that maintain indoor air into the most proper state according to use and purpose thereof. For example, such an air conditioner controls indoor air into a cold state in summer and controls indoor air into a warm state in winter. Furthermore, the air conditioner controls humidity of the indoor air and purifies the indoor air to become into a pleasant and clean state. Air conditioners may have a refrigeration cycle constituted by a compressor, a condenser, an expansion device, and an evaporator.

Such an air conditioner may be classified into a split type air conditioner in which indoor and outdoor units are separated from each other and an integral type air conditioner in which indoor and outdoor units are integrally coupled to each other as a single device, according to whether the indoor and outdoor units are separated from each other. Air conditioners are classified into a wall-mounted type air conditioner mounted on a wall, a frame type air conditioner, and a slim type air conditioner standing in the living room according to an installation method.

The air conditioner includes a fragrance generation device for containing fragrance within air. The fragrance generation device includes a solidified member and a receiving part for receiving the member. Here, air contacts the solidified member while the air flows to contain the fragrance therein.

In an air conditioner according to a related art, since a solidified member simply contacts air, an amount of fragrance contained in the air is less. Also, it is difficult to recognize a kind of fragrance contained in the air by a user.

EP 2 143 576 A1 discloses a fragrance dispenser which dispenses fragrant material into an air duct area by using a piezoelectrical pump. FR 2 867 424 A1 discloses a unit which enables selecting one of two fragrances and a level of the selected fragrance. EP 2 143 575 A1 discloses a fragrance dispenser, which pumps out fragrance mist upon a baffle plate located in an air flow passage by using a electrical motor. FR 2 946 256 A1 discloses a cartridge containing a volatile agent therein. EP 2 143 577 A1 discloses a fragrance dispenser with a fragrance cartridge and a fragrance discharge unit.

EP 1 686 326 A2 and US 5 957 771 A1 disclose free-standing air conditioners.

### SUMMARY

Embodiments provide an air conditioner in which an amount of fragrance contained in air can be increased, and a kind of fragrance can be confirmed by a user. According to the invention, an air conditioner comprises a case in which air flows; a discharge device disposed within the case to discharge a functional material from a container containing the functional material, wherein the container is receivable by the discharge device; a detection part detecting from the container information related to a kind of functional material in the container; and a display part displaying information based on the information detected by the detection part.

The container is preferably separable and independent from the air conditioner.

The discharge device may comprise a receiving part configured to receive the container containing the functional material, the receiving part contacting air to be discharged from the air conditioner; and a discharge mechanism being configured to discharge the functional material within the container.

The receiving part may be insertable into or withdrawable from the case.

The receiving part preferably comprises a receiving chamber in which the container is receivable, and wherein a portion of the discharge mechanism is disposed in the receiving chamber when the receiving part is in its inserted state.

Preferably, a portion of the discharge mechanism protrudes towards the receiving chamber when the receiving part is in its withdrawn state.

The air conditioner may further comprise a housing for receiving the receiving part, wherein the discharge mechanism is disposed in the housing.

The the discharge mechanism may contact the container when received in the receiving part.

The discharge mechanism may comprise at least one electrode for contacting the container.

The discharge mechanism may be inserted into the container while the container is received in the receiving part.

The discharge mechanism may be inserted into the container while the receiving part in which the container is received is inserted into the case or after the receiving part in which the container is received is inserted into the case.

The discharge mechanism may be configured to detect a pressure within the container while the discharge mechanism is inserted into the container and to detect the kind of functional material on the basis of the detected pressure.

The detection part may be configured to detect at least one of mounting of the container or the kind of functional material within the container, preferably by a signal inputted through an input part.

The detection part may comprise at least one of a weight sensor, an infrared sensor, a pressure sensor, a sensor for detecting a color, a reader for detecting at least one of an RFID tag, a QR code, and a bar code which are disposed on the container, and a hall sensor for detecting a magnet disposed in the container.

The air conditioner may further comprise a control part configured to control the display part for displaying information for informing the user that the container is received when the receiving of the container into the discharge device is detected.

The display part may be a light emitting part emitting light onto a panel.

The air conditioner may further comprise a control part configured to control the display part to display at least one of information related to the kind of functional material, effect information of the functional material, discharge time information of the functional material, and operation information for informing that the functional material is discharged.

The control part may be configured to control the display part to simultaneously or successively display at least two of the kind information, the effect information, the discharge time information, and the operation information.

The discharge time information of the functional material may be a time remaining until the functional material is completely discharged.

The remaining time may be countered only when the air conditioner is turned on.

The control part may be configured to control the display part to display a former remaining time when the air conditioner is turned on again after being turned off.

The discharge device may be operated only when the air conditioner is turned on.

The details of one or more embodiments are set forth in the accompanying drawings and the description below. Other features will be apparent from the description and drawings, and from the claims.

### BRIEF DESCRIPTION OF THE DRAWINGS

Fig. 1 is a perspective view of an air conditioner according to a first embodiment.
Fig. 2 is a front view of the air conditioner according to the first embodiment.
Fig. 3 is a perspective view of an air conditioner in which a discharge panel is opened according to the first embodiment.
Fig. 4 is a front view of the air conditioner in which the discharge panel is opened according to the first embodiment.
Fig. 5 is a cross-sectional view taken along line I-I' of Fig. 4.
Fig. 6 is a cross-sectional view taken along line II-II' of Fig. 4.
Fig. 7 is a view of the air conditioner in a state where an operation panel is moved in one direction according to the first embodiment.
Fig. 8 is a view of the air conditioner in a state where the operation panel is moved in the other direction according to the first embodiment.
Fig. 9 is a view illustrating a state in which a receiving part protrudes to the outside of the operation panel according to the first embodiment.
Fig. 10 is a horizontal sectional view of a discharge device according to the first embodiment.
Fig. 11 is an exploded perspective view of the discharge device according to the first embodiment.
Fig. 12 is a vertical sectional view of a discharge device according to the first embodiment.
Fig. 13 is a block diagram of the air conditioner according to the first embodiment.
Fig. 14 is a view illustrating a state in which the receiving part is inserted after being withdrawn from the air conditioner according to the first embodiment.
Fig. 15 is a view illustrating information displayed on the operation panel according to the first embodiment.
Fig. 16 is a block diagram of an air conditioner according to a second embodiment.

### DETAILED DESCRIPTION OF THE EMBODIMENTS

Exemplary embodiments of the present disclosure will be described below in more detail with reference to the accompanying drawings. It is also noted that like reference numerals denote like elements in appreciating the drawings even though the same elements are displayed on other drawings. Moreover, detailed descriptions related to well-known functions or configurations will be ruled out in order not to unnecessarily obscure subject matters of the present disclosure.

Also, in descriptions of the elements, terms "a first", "a second", etc and reference symbols "A", "B", "(A)", "(B)", etc may be used. These terms and reference symbols are used only to differentiate one element from the other element. Thus, the order of the elements corresponding to the terms and reference symbols given in the description is not limited thereto. In the following description, it will be understood that when an element is referred to as being "connected", "coupled", or "contact" another element, it can be directly connected or contact, or intervening elements may be also be "connected", "coupled" or "contact" between the elements.

Also, the idea of the present disclosure is not limited to a specific embodiment, but includes embodiments derived by combining the embodiments.

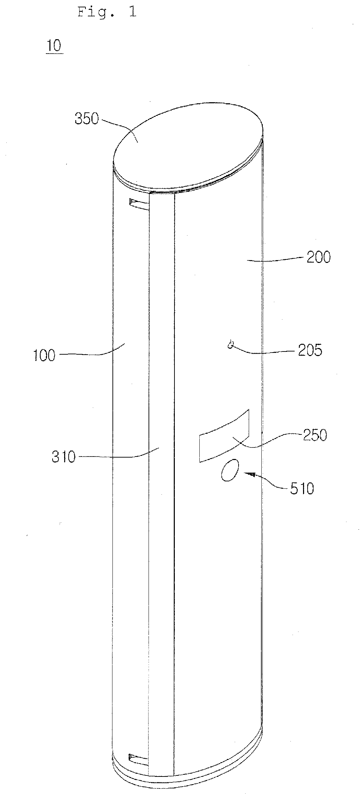

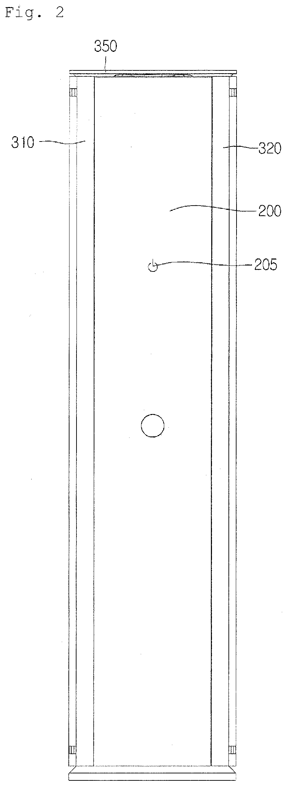

Fig. 1 is a perspective view of an air conditioner according to a first embodiment. Fig. 2 is a front view of the air conditioner according to the first embodiment.

Referring to Figs. 1 and 2, an air conditioner 10 according to the invention includes a case 100 defining an inner space, a movable operation panel disposed on a front side of the case 100, and movable discharge panels 310 and 320 disposed on at least one side of the operation panel 200.

The case 100 has a rounded outer appearance. Also, the case 100 may have an approximately oval shape on the whole. However, the current embodiment is not limited to the shape of the case 100. For example, the case 100 may have various shapes such as a circular, non-circular, polygonal, etc in section.

An outer appearance of a front or side surface of the air conditioner 10 may be defined by the operation panel 200 or the discharge panels 310 and 320. At least portions of the operation panel 200 and the discharge panels 310 and 320 may be rounded to correspond to that of the case 100.

Since the operation panel 200 defines a front outer appearance of the air conditioner 10, the operation panel 200 may be called a "front panel".

An input part 205 through which a user inputs a command is disposed on the operation panel 200. For example, the input part 205 may be a power input part for turning on/off a power of the air conditioner 10.

The discharge panels 310 and 320 include a first discharge panel 310 provided on one side of the operation panel 200 and a second discharge panel 320 provided on the other side of the operation panel 200. The first discharge panel 310 and the second discharge panel 320 may be moved in a direction toward or away from the operation panel 200.

An upper discharge device 350 for discharge air may be provided on an upper portion of the case 100. The upper discharge device 350 may be moved upward or downward.

When the upper discharge device 350 is not used, the upper discharge device 350 may be in a state (a state moved downward) received into the case 100. On the other hand, when the upper discharge device 350 is used, the upper discharge device 350 is moved upward to protrude (pop-up) upward from the case 100.

The upper discharge device 350 includes a housing 351 defining an outer appearance thereof. An upper discharge part 352 may be disposed on a front surface of the housing 351.

The case 100 may include a discharge device for discharging a functional material into the case 100. For example, the discharge device may be operated only when the air conditioner 10 is operated.

The discharge device may include a receiving part 510 for receiving a container (or a capsule) in which the functional material is contained. The receiving part 510 is movably disposed in the case 100. Also, the receiving part 510 may pass through the operation panel to protrude forward from the operation panel 200. Thus, a hole passing through the receiving part 510 may be defined in the operation panel 200. Also, a front surface of the receiving part 510 may define a portion of the outer appearance of the air conditioner 10. That is, the front surface of the receiving part 510 may be exposed to the outside of the air conditioner 10.

The operation panel 200 may include a display part 250 for displaying information of the functional material contained within the container received in the receiving part 510.

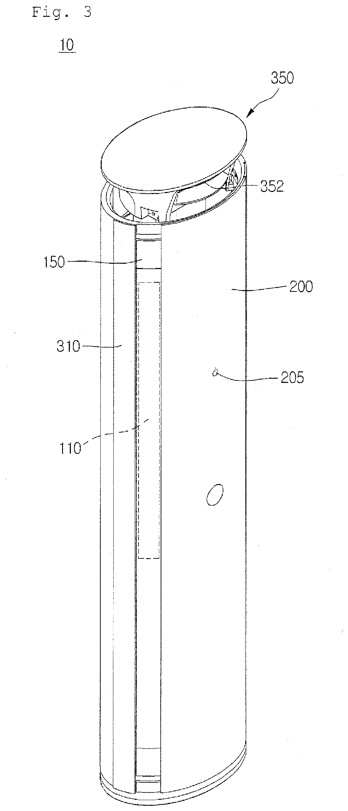

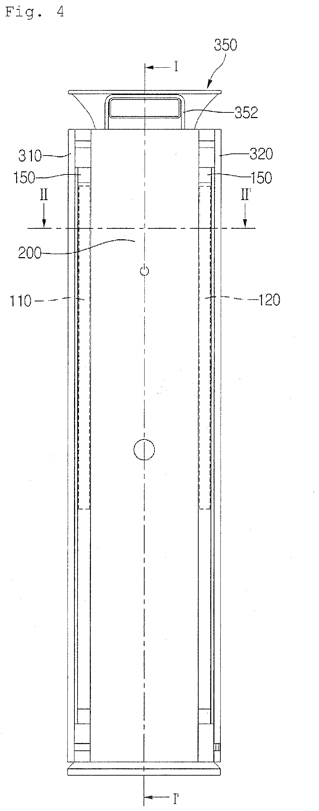

Fig. 3 is a perspective view of an air conditioner in which a discharge panel is opened according to the first embodiment. Fig. 4 is a front view of the air conditioner in which the discharge panel is opened according to the first embodiment.

Referring to Figs. 3 and 4, the air conditioner 10 according to the invention includes discharge parts 110 and 120 through which air is discharged. Also, the discharge pars 110 and 120 are disposed on both front surfaces of the case 100, respectively. A discharge grill for preventing foreign substances from being introduced or discharged may be disposed in each of both front sides of the case 100.

The discharge parts 110 and 120 include a first discharge part 110 disposed on one side of the operation panel 200 and a second discharge part 120 disposed on the other side of the operation panel 200. The first and second discharge parts 110 and 120 are disposed spaced apart from each other.

The operation panel 200 covers at least one portion of the first discharge part 110 and at least one portion of the second discharge part 120. In detail, the operation panel 200 is disposed between the first discharge part 110 and the second discharge part 120 to partition the first discharge part 110 from the second discharge part 120.

The first discharge panel 310 may selectively open or close the first discharge part 110. In detail, the first discharge panel 310 may be moved in a direction (a clockwise direction of Fig. 3) away from the operation panel 200. In this process, at least one portion of the first discharge part 110 may be opened.

On the other hand, the first discharge panel 310 may be moved in a direction (a counterclockwise direction of Fig. 3) toward the operation panel 200.. In this process, the first discharge part 110 may be covered.

The second discharge panel 320 may selectively open the second discharge part 120. In detail, the second discharge panel 320 may be moved in a direction (the counterclockwise direction of Fig. 3) away from the operation panel 200. In this process, at least one portion of the second discharge part 120 may be opened.

On the other hand, the second discharge panel 320 may be moved in a direction (the clockwise direction of Fig. 3) toward the operation panel 200. In this process, the second discharge part 120 may be covered.

When the first and second discharge parts 110 and 120 are covered by the first and second discharge panels 310 and 320, the air conditioner 10 may become to states of Figs. 1 and 2.

A discharge vane 150 may be movably disposed on each of the first and second discharge parts 110 and 120. The discharge vane 150 may be configured to adjust a flow direction of air discharged from the first and second discharge parts 110 and 120. The discharge vane 150 may be disposed within the operation panel 200 or the discharge panels 310 and 320.

When the first or second discharge panel 310 or 320 is opened, the discharge vane 150 is exposed to the outside. Also, when the discharge vane 150 is opened, air may be discharged to the outside through the first or second discharge part 110 or 120.

Hereinafter, an operation of the air conditioner according to the current embodiment will be simply described.

In a state where the air conditioner 10 is turned off, the air conditioner 10 may become to the states of Figs. 1 and 2.

That is, the operation panel 200 is disposed at a front central portion of the case 100. The first and second panels 310 and 320 covers the first and second discharge parts 110 and 120 on both sides of the operation panel 200, respectively. Here, the position of the operation panel 200 may be called a "central position" or a "first position".

When a user manipulates the input part 205 to turn on the air conditioner 10, each of the first and second discharge panels 310 and 320 may be moved in the direction away from the operation panel 200. For example, the first discharge panel 310 may be moved in a clockwise direction, and the second discharge panel 320 may be moved in a counterclockwise direction.

When the first and second discharge panels 310 and 320 are opened, the discharge vane 150 is exposed to the outside. Then, the discharge vane 150 is rotated or moved to open the first discharge part 110 or the second discharge part 120. That is, air may be discharged through both sides of the operation panel 200.

Here, a flow direction of air discharged from the first and second discharge parts 110 and 120 may be adjusted according to a rotated angle or moving distance of the discharge vane 150.

When the input part 205 is manipulated while the air conditioner 10 is operated, the air conditioner 10 may be turned off. When the power is turned off, the discharge vane 150 may be disposed to cover the first and second discharge parts 110 and 120.

Also, the first and second discharge panels 310 and 320 are moved toward the operation panel 200 to cover the first and second discharge parts 110 and 120. For example, the first discharge panel 310 may be moved in a counterclockwise direction, and the second discharge panel 320 may be moved in a clockwise direction.

When the first and second discharge panels 310 and 320 are closed, as shown in Fig. 2, the first and second panels 310 and 320 may be disposed to approximately contact both sides of the operation panel 200.

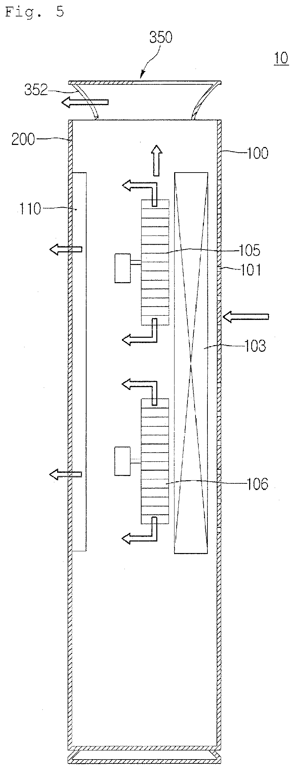

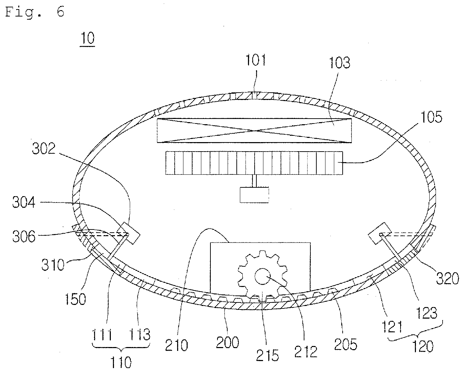

Fig. 5 is a cross-sectional view taken along line I-I' of Fig. 4. Fig. 6 is a cross-sectional view taken along line II-II' of Fig. 4.

Referring to Figs. 5 and 6, the air conditioner 10 according to the first embodiment includes a suction part 101 through which air is suctioned and the plurality of discharge parts 110, 120, 352 through which air is discharged.

The suction part 101 is disposed in a rear surface of the case 100 to suction air. Also, a heat exchanger 103 and blower fans 105 and 106 are disposed on a front side of the suction part 101. The blower fans 105 and 106 may include a first fan 105 and a second fan 106 disposed under the first fan 105.

The plurality of discharge parts 110, 120, and 352 include the first and second discharge parts 110 and 120 respectively disposed at left and right sides of the operation panel 200 and an upper discharge part 352 disposed on the upper discharge device. The upper discharge part 352 is opened when the upper discharge device 350 is moved upward from the case 100. Also, the upper discharge part 352 is closed when the upper discharge device 350 is received into the case 100.

Each of the discharge parts may be opened or closed together with each other or independently opened or closed.

When the blower fans 105 and 106 are operated, air is introduced into the case 100 through the suction part 101 to pass through the heat exchanger 103. Then, the air may be branched into the plurality of discharge parts 110, 120, and 352 while passing through the first and second fans 105 and 106 and discharged.

For example, the air passing through the first fan 105 may flow into the upper discharge part 352 and the first and second discharge parts 110 and 120. Also, the air passing through the second fan 106 may flow into the first discharge part 110 or the second discharge part 120.

The air conditioner 10 may further include a driving device for moving the operation panel 200 and the discharge panels 310 and 320.

The driving device includes a driving motor 210 for generating a driving force for moving the operation panel 200, a pinion gear 215 rotated by the operation of the driving motor 210, and a rack gear 205 linked with the pinion gear 215.

The driving motor 210 is disposed at a rear side of the operation panel and includes a motor shaft 212. The pinion gear 215 is connected to the motor shaft 212 and rotated together with the motor shaft 212. Also, the rack gear 205 may be disposed on a rear surface of the operation panel 200.

The driving motor may be a bidirectionally rotatable motor.

When the driving motor 210 is rotated in one direction, the pinion gear 215 may be rotated to correspond to the rotation of the driving motor 210. Then, the rack gear 205 may be linked with the pinion gear 215 and thus moved in a clockwise direction (a left side when viewed from a front surface of Fig. 2).

Thus, the operation panel 200 may be operated to cover at least one portion of the first discharge part 110. Here, the first discharge panel 310 may be in the opened state.

On the other hand, when the driving motor 210 is rotated in the other direction, the pinion gear 215 may be rotated to correspond to the rotation of the driving motor 210. Then, the rack gear 205 may be linked with the pinion gear 215 and thus moved in a counterclockwise direction (a right side when viewed from the front surface of Fig. 2).

Thus, the operation panel 200 may be operated to cover at least one portion of the second discharge part 120. Here, the second discharge panel 320 may be in the opened state.

The driving device includes a discharge motor 302 for generating a driving force for moving the discharge panels 310 and 320 and a power transmission member 306 rotated according to an operation of the discharge motor 302.

The power transmission member 306 may be connected to a motor shaft 304 of the discharge motor 302 and rotated in a clockwise or counterclockwise direction. The power transmission member 306 may be a link member. The power transmission member 306 may be coupled to a rear surface of each of the discharge panels 310 and 320.

The discharge motor 302 and the power transmission member 306 may be disposed on both inner sides of the case 100 to move the first and second discharge panels 310 and 320, respectively. The discharge motor 302 may be a bidirectionally rotatable motor.

In the operation of the first discharge panel 310, when the discharge motor 302 and the motor shaft 304 are rotated in one direction, the power transmission member 306 is rotated in a clockwise direction (a dotted line of Fig. 6). Thus, the first discharge panel 310 is operated to open the first discharge part 110.

On the other hand, in a state where the first discharge panel 310 is opened, when the discharge motor 302 and the motor shaft 304 are rotated in the other direction, the power transmission member 306 is rotated in the counterclockwise direction (a solid line of Fig. 6). Thus, the first discharge panel 310 is operated to close at least one portion of the first discharge part 110.

In the operation of the second discharge panel 320, when the power transmission member 306 is rotated in the counterclockwise direction, the second discharge panel 320 is operated to open the second discharge part 120 (the dotted line of Fig. 6).

On the other hand, in the state where the second discharge panel 320 is opened, when the power transmission member 306 is rotated in the clockwise direction, the second discharge panel 320 is operated to close at least one portion of the second discharge part 120 (the solid line of Fig. 6).

The first discharge part 110 includes a first discharge region and a second discharge region which are selectively covered. The first and second discharge regions 111 and 113 define a portion region and a region except the portion region of the first discharge part 110.

The discharge vane 150 is disposed in a front side of each of the first and second discharge regions 111 and 113. Thus, each of the first and second discharge regions 111 and 113 may be understood as a region opened or closed by the discharge vane 150, i.e., a region corresponding to the discharge vane 150.

Similarly, the second discharge part 120 includes a third discharge region 121 and a fourth discharge region 123. The discharge vane 150 is disposed in a front side of each of the third and fourth discharge regions 121 and 123.

As shown in Figs. 1 and 2, in a state where all of the first and second discharge parts 110 and 120 are closed, the first area 111 is covered by the first discharge panel 310, and the second area 113 is covered by the operation panel 200.

Also, the third discharge area 121 is covered by the operation panel 200, and the fourth discharge area 123 is covered by the second discharge panel 320.

Here, the second and third discharge areas 113 and 121 may be spaced apart from each other. Also, the second and third discharge areas 113 and 121 may be covered at the same time by the operation panel 200. The second discharge area 113 and the third discharge area 121 may be understood as central areas of the first and second discharge parts 110 and 120, respectively.

In this state, when the first discharge panel 310 is opened, a portion of the first discharge part 110, the first discharge area 111 is exposed to the outside. Also, when the second discharge panel 320 is opened, a portion of the second discharge part 120, i.e., the fourth discharge area 123 is exposed to the outside.

When the discharge vane 150 corresponding to the first discharge area 111 and the discharge vane 150 corresponding to the fourth discharge area 123 are opened, air is discharged through the corresponding discharge areas 111 and 123.

The operation panel 200 is disposed at a front central position of the case 100, i.e., the first position to cover the second and third discharge areas 113 and 121. Thus, the discharge of air through the second and third discharge areas 113 and 121 may be restricted. Thus, air may be discharged through the first and fourth discharge areas 111 and 123.

As a result, the air may be discharged through the opened discharge areas of both sides of the operation panel 200 in both side directions. That is to say, the opened areas of the discharge parts 110 and 120 may be disposed on both sides of the operation panel 200.

In summary, since the areas through which the air is actually discharged among all of the discharge areas 111, 113, 121, and 123 may be the portion areas 111 and 123, an air discharge area of the first and second discharge parts 110 and 120 may be less than the sum of areas of all of the discharge parts 110 and 120.

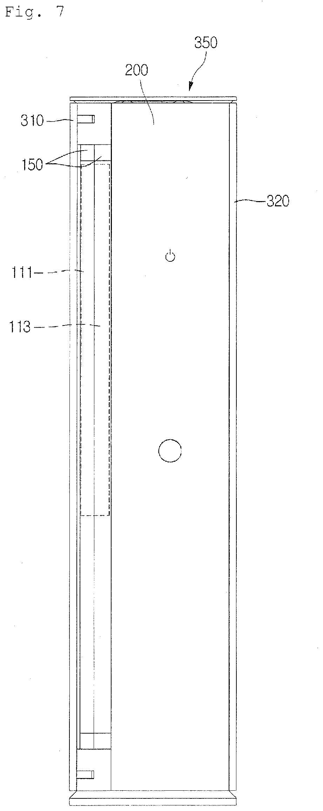

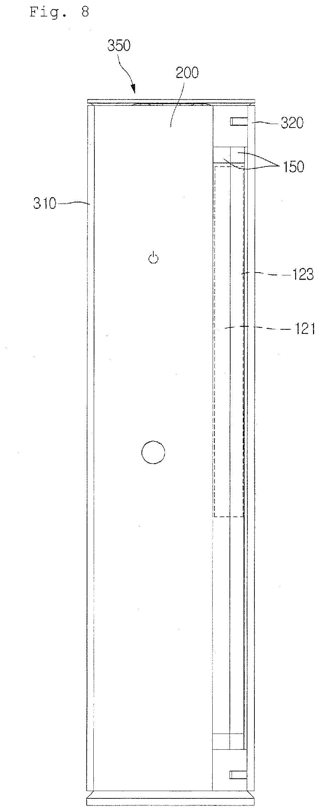

Fig. 7 is a view of the air conditioner in a state where an operation panel is moved in one direction according to the first embodiment. Fig. 8 is a view of the air conditioner in a state where the operation panel is moved in the other direction according to the first embodiment.

Referring to Fig. 7, in a state of the first position shown in Fig. 4, the operation panel 200 may be moved toward the second discharge part 120 from the first discharge part 110, i.e., in a right direction or counterclockwise direction. Here, a position of the operation panel 200 may be called a "right position" of a "second position".

As described above, when the operation panel 200 is moved to the second position, the second discharge area 113 is opened.

In detail, the second discharge area 113 may be exposed to the outside. Also, the discharge vane 150 corresponding to the second discharge area 113 may be opened to discharge air from the second discharge area 113. As a result, air may be discharged through the first and second discharge areas 111 and 113, i.e., the whole area of the first discharge part 110.

In summary, the opened area of the first discharge part 110 is increased according to the movement of the operation panel 200, and thus the amount of air discharged through the first discharge part 110 is increased.

As the operation panel 200 is moved to the second position, the fourth discharge area 123 is covered by the operation panel 200. As a result, the third and fourth discharge areas 121 and 123, i.e., the whole of the second discharge part 120 may be closed, and thus, the discharge of air through the second discharge part 120 may be restricted.

In summary, the opened area of the second discharge part 120 may be increased according to the movement of the operation panel 200, and thus the amount of air discharged through the second discharge part 120 may be increased. Thus, air may be concentratedly discharged toward a side of the operation panel 200.

As described above, since air is concentratedly discharged toward a side of the air conditioner 10 according to a position of the operation panel 200, the personalized operation of the air conditioner 10 may be allowable according to the installation position of the air conditioner 10 or the position of the user.

However, the opened area, through which air is discharged, of the whole area of the first and second discharge parts 110 and 120 may be constant regardless of the first or second position of the operation panel 200.

When the operation panel 200 is disposed at the second position, an actual air discharge area of the whole discharge areas 111, 113, 121, and 123 may be restricted to portion areas 121 and 123. Thus, the air discharge area of the first and second discharge parts 110 and 120 may be less than the whole area of the first and second discharge parts 110 and 120.

Referring to Fig. 8, in a state of the first position shown in Fig. 4, the operation panel 200 may be moved toward the first discharge part 120 from the second discharge part 120, i.e., in a left direction or a clockwise direction. Here, a position of the operation panel 200 may be called a "left position" of a "third position".

As described above, when the operation panel 200 is moved to the third position, the third discharge area 121 is opened.

In detail, the third discharge area 121 may be exposed to the outside. Also, the discharge vane 150 corresponding to the third discharge area 121 may be opened to discharge air from the third discharge area 121. As a result, air may be discharged through the third and fourth discharge areas 121 and 123, i.e., the whole area of the second discharge part 110.

In summary, the opened area of the second discharge part 120 is increased according to the movement of the operation panel 200, and thus the amount of air discharged through the second discharge part 120 is increased.

As the operation panel 200 is moved to the third position, the first discharge area 111 is covered by the operation panel 200. As a result, the first and second discharge areas 111 and 113, i.e., the whole of the second discharge part 120 may be closed, and thus, the discharge of air through the second discharge part 120 may be restricted.

As described above, the opened area of the first discharge part 110 may be increased according to the movement of the operation panel 200, and thus the amount of air discharged through the first discharge part 110 may be increased. Thus, air may be concentratedly discharged toward the other side of the operation panel 200.

As described above, since air is concentratedly discharged toward the other side of the air conditioner 10 according to a position of the operation panel 200, the personalized operation of the air conditioner 10 may be allowable according to the installation position of the air conditioner 10 or the position of the user.

However, the opened area, through which air is discharged, of the whole area of the first and second discharge parts 110,120 and 120 may be constant regardless of the first or third position of the operation panel 200.

When the operation panel 200 is disposed at the third position, an actual air discharge area of the whole discharge areas 121, 113, 121, and 123 may be restricted to portion areas 121 and 123. Thus, the air discharge area of the first and second discharge parts 110 and 120 may be less than the whole area of the first and second discharge parts 110 and 120.

In the current embodiment, although the operation panel 200 is moved from the first position to the second position or from the first position to the third position, the present disclosure is not limited thereto. For example, the operation panel 200 may be moved from the second position to the first position or from the third position to the first position.

Also, the operation panel 200 may be moved from the second position to the third position or from the third position to the second position.

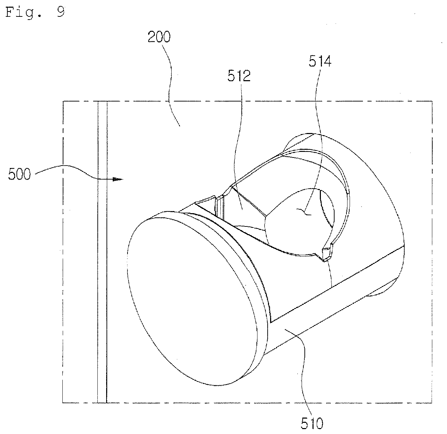

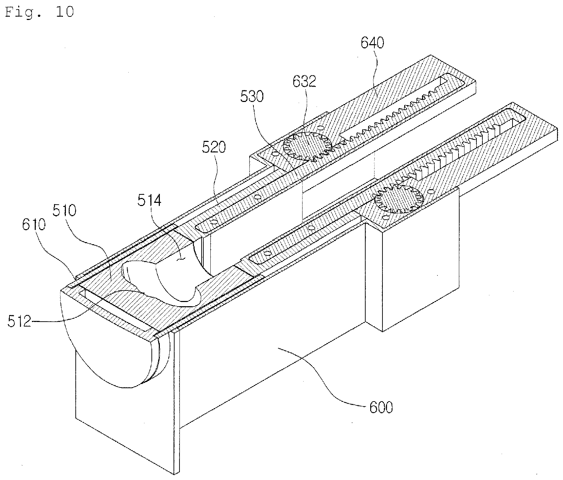

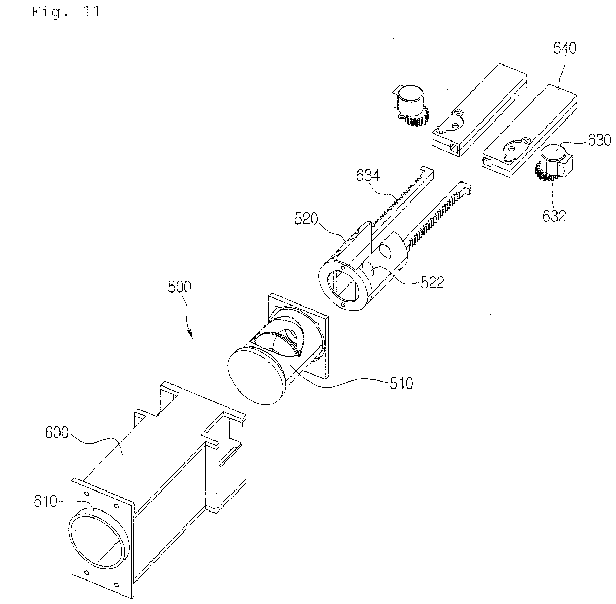

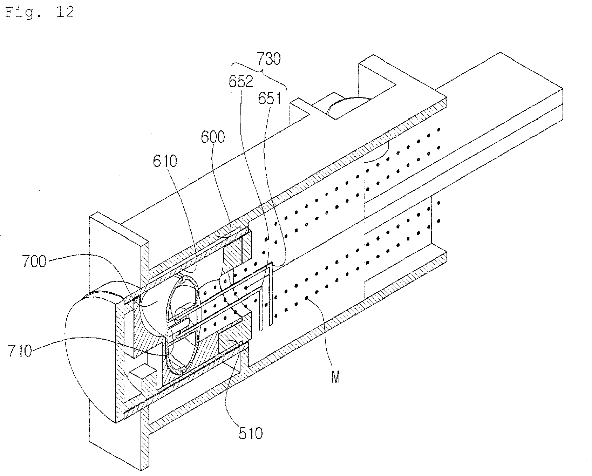

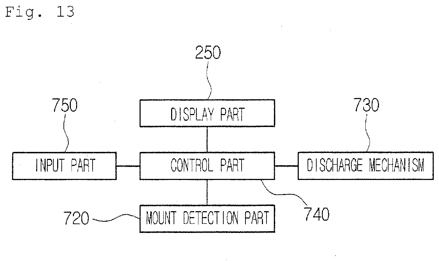

Fig. 9 is a view illustrating a state in which a receiving part protrudes to the outside of the operation panel according to the first embodiment. Fig. 10 is a horizontal sectional view of a discharge device according to the first embodiment. Fig. 11 is an exploded perspective view of the discharge device according to the first embodiment. Fig. 12 is a vertical sectional view of a discharge device according to the first embodiment. Fig. 13 is a block diagram of the air conditioner according to the first embodiment.

Referring to Figs. 9 to 13, a discharge device 500 of the air conditioner according to the invention includes a housing fixed to the operation panel 200, a receiving part 510 movably disposed within the housing 600, a motor 630 generating a power for moving the receiving part 510, and a power transmission part for transmitting the power of the motor 630 into the receiving part 510.

In the current embodiment, the motor 630 and the power transmission part may be called a "moving mechanism" for moving the receiving part 510.

Also, the discharge device 500 may include a discharge mechanism 730 for discharging a functional material within a container 700 received in the receiving part 510 from the container 700 and a mount detection part 720 for detecting whether the container 700 is mounted on the receiving part 510.

The receiving part 510 includes a chamber 512 for receiving the container 700. The chamber 512 may have a shape corresponding to that of the container 700. Also, the receiving part 510 has a passage 514 through which the functional material discharged from the container 700 flows. The passage 514 communicates with an air passage within the case 100. That is, the receiving part 510 may contact air within the case 100. Thus, the functional material discharged from the container 700 is discharged to the outside of the air conditioner 10 together with the air within the air passage.

The mount detection part 720 may be provided in the chamber 512. In the current embodiment, the mount detection part 720 may include at least one of a reader for detecting at least one of an RFID tag, a QR code, and a bar code disposed on the container 700, a sensor for detecting a color of the container 700, a hall sensor for detecting a magnet disposed on the container 700, a weight sensor for detecting a weight, an infrared sensor, and a pressure sensor.

If the mount detection part 720 is the reader, the mount detection part 720 may recognize the mounting of the container 700 as well as various information (referred to as container-related information) related to a kind of functional material contained in the container 700.

When at least one portion of the container 700 has a different color according to a kind of functional material contained in the container 700, the mount detection part 720 may detect a color of the container 700 to recognize a kind of functional material.

When the mount detection part 720 is the hall sensor, and a plurality of hall sensors are provided in the receiving part 510, the mount detection part 720 may recognize the mounting of the container 700 as well as various information such as a kind of functional material contained in the container 700. That is, the magnet disposed in the container may be changed in position according to a kind of functional material. Thus, information related to a kind of functional material may be recognized according to which one of the plurality of hall sensors detects the magnet.

Alternatively, the user may input the mounting of the container through an input part 750 or input a kind of functional material contained in the container 700.

The power transmission part may include a pinion gear 632 connected to the motor 630 and a rack gear 634 engaged with the pinion gear 634. The rack gear 634 is connected to a connection member 520, and the connection member 520 is connected to the receiving part 510. Here, since the connection member 520 is connected to the rack gear 634 to transmit a power of the motor 630, the connection member 520 may be one component of the power transmission part.

For another example, the rack gear 634 may be connected to the receiving part 510 or disposed on the receiving part 510.

The connection member 520 may have a passage through which the functional material flows. Also, the connection member 520 may have a coupling hole through which a coupling part passes so that the connection member 520 is coupled to the rack gear 634.

The motor 630 may be seated on a motor support 640. The motor support 640 may be fixed to the housing 600. The motor support 600 may have a seat part for seating the pinion gear 632 and the rack gear 634. Thus, the seat part may guide the movement of the rack gear 634.

A guide part 610 for guiding the movement of the receiving part 510 may be provided in the housing 600. The guide part 610 may have a hollow. Thus, the receiving part 510 may be moved within the guide part 610.

The discharge mechanism 730 for discharging the functional material from the container may be disposed within the housing 600.

In detail, the container 700 may be pitted or torn by an external force. Alternatively, the container 700 may have a portion which is capable of being pitted or torn by an external force. The discharge mechanism 730 may pass through a portion of the container 700 and then be inserted into the container 700.

A release part 710 in which the functional material (for example, liquid) is immersed is provided within the container 700. The release part 710 may be a porous medium.

The discharge mechanism 730 may include a pair of electrodes 651 and 652.

The pair of electrodes 651 and 652 may be disposed in the housing 600 or the receiving part 510. When the discharge mechanism 730 is disposed in the receiving part 510, ends of the pair of electrodes 651 and 652 may protrude from the receiving chamber 512. In this case, when the container 700 is mounted on the receiving part 510, the discharge mechanism 730 may be inserted into the container 700.

The pair of electrodes 651 and 652 may contact the release part 710. The pair of electrodes 651 and 652 may be connected to a power supply part (not shown), and the power supply part may be controlled by a control part 740.

Also, the control part 740 may control at least one of a light emitting part, a display part, and the motor constituting the moving mechanism.

The discharge mechanism 730 may be disposed on the receiving chamber 512 in a state where the receiving part 510 is inserted into the case 100. That is, since the receiving part 510 has a passage 514, the discharge mechanism 730 may be disposed on the receiving chamber 512 by the passage 514.

When the discharge mechanism 730 is inserted into the container 700, a pressure applied to the discharge mechanism 730 may be detected to recognize information with respect to a kind of functional material within the container 700. That is, when a pressure within the container 700 is varied according to a kind of functional material within the container 700, the kind of functional material contained in the container 700 may be determined by a pressure applied to the discharge mechanism 730 while the discharge mechanism 730 passes through the container 700.

Hereinafter, an operation of the discharge device will be described.

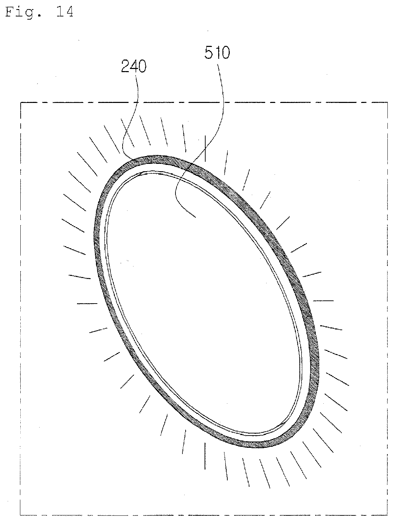

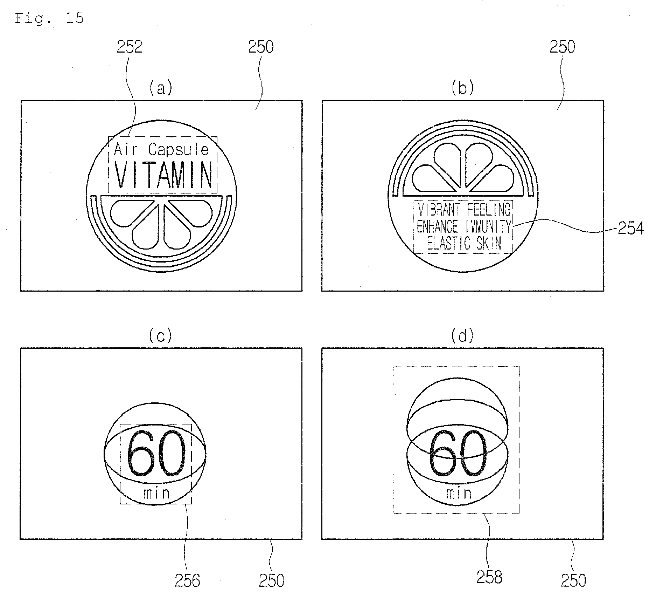

Fig. 14 is a view illustrating a state in which the receiving part is inserted after being withdrawn from the air conditioner according to the first embodiment. Fig. 15 is a view illustrating information displayed on the operation panel according to the first embodiment.

Referring to Figs. 14 and 15, when a withdrawal command of the receiving part 510 is inputted through the input part 750, the receiving part 510 may be withdrawn toward a front side of the operation panel 200 by the moving mechanism as shown in Fig. 9.

Then, the user receives the container 700 containing the functional material into the receiving part 510. The container 700 may be a disposable container. Since the user may receive the container 700 into the receiving part 510, one functional material of various kinds of functional materials may be contained in air.

When the container 700 is received into the receiving part 510, the mount detection part 730 detects the mounting of the container 700. Thus, the receiving part 510 is inserted into the case 100 by the moving mechanism.

For another example, when a predetermined time elapses after the receiving part 510 is withdrawn to the outside of the case 100, the receiving part 510 may be automatically inserted into the case 100. Alternatively, when the mounting of the container 700 is not detected for a predetermined time after the receiving part 510 is withdrawn to the outside of the case 100, the receiving part 510 may be automatically inserted into the case 100.

For another example, when an insertion command of the receiving part 510 is inputted through the input part 750, the receiving part 510 may be inserted into the case 100.

In the current embodiment, the input part 750 may be disposed in the operation panel 200, a remote controller for remotely controlling the air conditioner 10, or a front surface of the receiving part 510.

When the input part 750 is disposed in the receiving part 510, the withdrawal command or the insertion command of the receiving part 510 may be inputted in a touch method. Alternatively, a withdrawal button or an insertion button as the input part may be provided in the receiving part 510. Alternatively, the withdrawal command and the insertion command may be successively inputted using one button.

When the receiving part 510 is withdrawn from the case 100, information for requiring the mounting of the . container 700 or information for guiding the mounting method may be displayed on the display part 250.

Also, when the receiving part 510 is inserted into the case 100, in a case where the container 700 is not mounted within the receiving part 510, information for informing that the container 700 is not mounted may be disposed on the display part 250. In this case, the user may input a command for withdrawing the receiving part 510 again.

When the receiving part 510 is inserted into the case 100 in a state where the container 700 is received into the receiving part 510, information for informing that the container 700 is inserted into the surrounding of the receiving part 510 of the operation panel 200 or information for informing that the receiving part 510 is inserted may be displayed. For example, as shown in Fig. 9, light may be emitted from the operation panel 200. That is, the light emitting part (a display part for displaying information) for emitting light may be disposed in the operation panel 200 or the case 100. In the state where the container 700 is received into the receiving part 510, when the receiving part 510 is inserted into the case 100, the light emitting part may be turned on to emit light onto the operation panel 200.

For example, the receiving part 510 may have a circular front surface, and thus, the light emitted onto the operation panel 200 may be seen in a ring shape on the operation panel 200. Here, the front surface of the receiving part 510 may be disposed within the information having the ring shape.

For another example, when the receiving part 700 is inserted into the case 100 in the state where the container 700 is not mounted within the receiving part 510, the information having the ring shape may be displayed on the operation panel 200. Here, the information having the ring shape may be the same as or different from the information having the ring shape displayed when the receiving part 510 is inserted into the case 100 in the state where the container 700 is mounted within the receiving part 510. That is, when the receiving part 510 is inserted into the case 100 in the state where the container 700 is mounted within the receiving part 510, first color information may be displayed on the operation panel 200. On the other hand, when the receiving part 510 is inserted into the case 100 in the state where the container 700 is not mounted within the receiving part 510, second color information may be displayed on the operation panel 200.

The present disclosure is not limited to a kind and shape of information displayed on the operation panel 200. That is, the information displayed on the operation panel 200 may include a texture, an image, an icon, and the like displayed on the display part 250 provided in the operation panel 200 in addition to the light. Alternatively, when the receiving part 510 is inserted into the case 100 in the state where the container 700 is mounted or not mounted, information for informing this may be outputted as voice.

When the receiving part 510 is inserted into the case 100 in the state where the container 700 is mounted, the discharge mechanism 730 passes through the passage 514 of the receiving part 510 and then is inserted into the container 700 mounted within the receiving chamber 512. That is, the pair of electrodes 651 and 652 passes through the container 700 and then contacts the release part 710 within the container 700.

For another example, the discharge mechanism 730 is movably disposed within the housing 600, the receiving part 510 in which the container 700 is mounted is inserted into the case 100, and then, the discharge mechanism 730 is moved and inserted into the container 700.

Also, a voltage may be applied into the pair of electrodes 651 and 652 under the control of the control part 740. The functional material M within the container 700 may exist in an ion state. When a voltage is applied into the pair of electrodes 651 and 652, the functional material M charged in the release part 710 may be atomized, and thus, the atomized functional material M together with air may be discharged to the outside of the air conditioner 10 by an air current within the case 100.

If the functional material contained in the container 700 is nonconductive liquid, since ions serving as charges do not exist almost, it may be difficult to charge the functional material. In this case, an electrode may be injected into the container 700.

In detail, an electrode needle and a ground pole spaced apart from the electrode needle may be disposed within the housing 600 or the receiving chamber 512 of the receiving part 510. Also, when the container 700 is received into the receiving part 510, the electrode needle contacts the release part 710 within the container 700, and the ground pole is disposed on a side of the outside of the container 700. In this state, when a voltage is applied into the electrode needle, electrons are released from the electrode needle to charge the liquid around the electrode needle. The charged fluid is atomized at an end of the release part 710 by an electric force greater than a surface tension of the fluid, and then the atomized fluid is discharged to the outside. Here, to release the atomized material to the outside, the end of the release part 710 may be exposed to the outside of the container 700.

Referring to Fig. 15, when the container 700 is mounted within the receiving part 510, and the detection part recognizes information related to the container 700, the control part controls the display part 250 so that the display part 250 displays information based on the information detected by the detection part.

For example, information 252 related to a kind of functional material contained in the container 700 may be displayed on the display part 250. Then, effect information 254 of the recognized functional material may be displayed on the display part 250.

Then, time information 256 taken when the functional material is discharged from the container 700 or operation time information of the air conditioner in the case where the functional material together with air is discharged from the air conditioner may be displayed on the display part 250.

Then, operation information 256 for informing that the discharge mechanism 730 may be operated to discharge the functional material from the container 700 may be displayed.

For example, referring to Fig. 15A, a circular container shape may be displayed on the display part 250, and information 252 related to a kind of functional material may be displayed within the container shape. For example, referring to Fig. 15B, effect information 254 of the functional material may be displayed within the container shape. Here, an area on which the information 252 related to the kind of functional material is displayed and an area on which the effect information 254 of the functional material is displayed may be different from each other.

For another example, the kind information 252 and the effect information 254 may be displayed at the same time.

Referring to Fig. 15C, the time information 256 may be displayed within the circular container shape. Referring to Fig. 15D, the operation information 258 may be displayed as a shape in which a circular or globular-shaped container is divided into two parts. Also, when the operation information 258 is displayed, the time information 256 may be displayed together. Also, as a time elapses, a time at which the time information 256 is displayed may be reduced. That is, the time information 256 may be time information remaining until the functional material is completely discharged. The remaining time may be counted only when the air conditioner is turned on. That is, when the air conditioner is turned on and then off in a state where the remaining time is displayed on the display part 250, a former remaining time may be displayed on the display part 250.

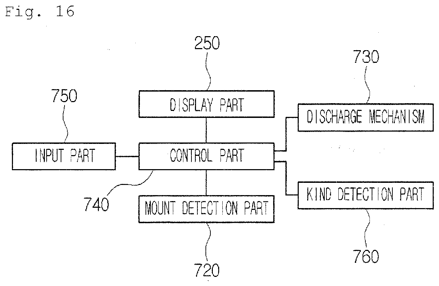

Fig. 16 is a block diagram of an air conditioner according to a second embodiment.

The second embodiment is equal to the first embodiment except that a kind detection part for recognizing kind information of a function material is further provided. Thus, only specific portions of the current embodiment will be described below.

Referring to Fig. 16, a discharge device according to the second embodiment may include a mount detection part 720 for detecting the mounting of a container 700 and a kind detection part 760 for detecting a kind of functional material within the container 700.

The kind detection part 760 may include least one of a reader for detecting at least one of an RFID tag, a QR code, and a bar code disposed on the container 700, a sensor for detecting a color of the container 700, and a hall sensor for detecting a magnet disposed on the container 700.

If the mount detection part 720 is the reader, the kind detection part 760 may recognize the mounting of the container 700 as well as various information such as the kind information of the functional material contained in the container 700.

When at least one portion of the container 700 has a different color according to a kind of functional material contained in the container 700, the kind detection part 760 may detect a color of the container 700 to recognize a kind of functional material.

When the kind detection part 760 is the hall sensor, and a plurality of hall sensors are provided in the receiving part 510, the kind detection part 760 may recognize a kind of function material contained in the container 700. That is, the magnet disposed in the container may be changed in position according to a kind of functional material. Thus, information related to a kind of functional material may be recognized according to which one of the plurality of hall sensors detects the magnet.

Although the discharge device is installed in the air conditioner in the embodiments, the present disclosure is not limited thereto. For example, the discharge device may be applied to any product in which air can flow, and a functional material together with the air can be discharged to the outside. That is, the discharge device may be applied to various products such as an air cleaner, a dehumidifier, and the like.

The above-described display part may include a display panel, the light emitting part, an image irradiation part for irradiating an image, and the like.

For example, an image irradiated from the image irradiation part may be irradiated onto the operation panel to display information on the operation panel. Of cause, in a case where a separate operation panel is not provided, the information may be displayed on the case.

According to the proposed embodiments, the container containing the functional material may be mounted within the receiving part, and the functional material may be discharged from the container. Thus, an amount of functional material contained in the air discharged from the air conditioner may be increased.

Also, since the information related to the container is displayed on the display part, the user may easily confirm whether the container exists and a kind of functional material.

## Claims

1. An air conditioner comprising a case (100) defining an inner space, in which air flows during use of the air conditioner; a first and a second discharge part (110, 120) disposed to be spaced apart from each other on the front of the case (100), and through which air is discharged;
a movable operation panel (200) disposed on a front side of the case (100) between the first and the second discharge parts and covering at least one portion of each of the discharge parts (110, 120);
movable discharge panels (310, 320) disposed on at least one side of the movable operation panel (200);
a discharge device (500) disposed within the case for discharging a functional material from a container (700) containing the functional material;
a detection part (720) detecting from the container information related to a kind of functional material in the container; and
a display part (250) displaying information based on the information detected by the detection part,
wherein the discharge device(500) comprises:
a housing (600) fixed to the movable operation panel (200);
a receiving part (510) movably disposed within the housing (600) ;
a motor (630) generating a power for moving the receiving part (510); and
a power transmission part for transmitting the power of the motor (630) to the receiving part (510),
wherein the receiving part (510) comprises:
a chamber (512) for receiving the container (700); and
a passage (514) communicating with an air passage within the case (100), and
wherein the functional material discharged from the container (700) passes through the passage (514) and is discharged to the outside from the discharge parts (110,120) together with air in the inner space.

2. The air conditioner according to claim 1, wherein the discharge device (500) further comprises a discharge mechanism (730) being configured to discharge the functional material from the container.

3. The air conditioner according to claim 2, wherein the receiving part (510) is insertable into or withdrawable from the case (100).

4. The air conditioner according to claim 2 or 3,
wherein a portion of the discharge mechanism (730) is disposed in the receiving chamber when the receiving part (510) is in its inserted state.

5. The air conditioner according to claim 4, wherein a portion of the discharge mechanism (730) protrudes towards the receiving chamber (512) when the receiving part (510) is in its withdrawn state.

6. The air conditioner according to claim 4, wherein the discharge mechanism (730) is disposed in the housing.

7. The air conditioner according to any one of claims 2 to 6, wherein the discharge mechanism (730) contacts the container (700) when received in the receiving part (510).

8. The air conditioner according to any one of claims 2 to 7, wherein the discharge mechanism (730) comprises at least one electrode (651, 652) for contacting the container (700).

9. The air conditioner according to claim 7 or 8, wherein the discharge mechanism (730) is inserted into the container (700) while the container (700) is received in the receiving part (510) .

10. The air conditioner according to claim 7, 8, or 9, wherein the discharge mechanism (730) is configured to be inserted into the container (700) while the receiving part (510) in which the container (700) is received is inserted into the case (100) or after the receiving part (510) in which the container is received is inserted into the case (100).

11. The air conditioner according to any one of claims 2 to 10, wherein the discharge mechanism (730) is configured to detect a pressure within the container (700) while the discharge mechanism (730) is inserted into the container (700) and to detect the kind of functional material on the basis of the detected pressure.

12. The air conditioner according to any one of claims 1 to 11, wherein the detection part (720) is configured to detect at least one of mounting of the container (700) or the kind of functional material within the container (700), preferably by a signal inputted through an input part.

13. The air conditioner according to any one of claims 1 to 12, further comprising a control part (740) configured to control the display part (250) for displaying information for informing the user that the container (700) is received when the receiving of the container into the discharge device is detected.

14. The air conditioner according to any one of claims 1 to 13, further comprising a control part (740) configured to control the display part (250) to display at least one of information related to the kind of functional material, effect information of the functional material, discharge time information of the functional material, and operation information for informing that the functional material is discharged.

15. The air conditioner according to claim 14, wherein the control part (740) is configured to control the display part (250) to simultaneously or successively display at least two of the kind information, the effect information, the discharge time information, and the operation information.

## Patentansprüche

1. Klimaanlage, die aufweist:
ein Außengehäuse (100), das einen Innenraum definiert, in dem während der Verwendung der Klimaanlage dem Luft strömt;
einen ersten und einen zweiten Ausstoßteil (110, 120), die so auf der Vorderseite des Außengehäuses (100) angeordnet sind, dass sie voneinander beabstandet sind, und durch die Luft ausgestoßen wird;
ein bewegliches Bedienungsfeld (200), das auf einer Vorderseite des Außengehäuses (100) zwischen dem ersten und dem zweiten Ausstoßteil angeordnet ist und mindestens einen Abschnitt von jedem der Ausstoßteile (110, 120) bedeckt;
bewegliche Ausstoßplatten (310, 320), die auf mindestens einer Seite des beweglichen Bedienungsfelds (200) angeordnet sind;
eine Ausstoßvorrichtung (500), die innerhalb des Außengehäuses zum Ausstoßen eines Funktionsmaterials aus einem Behälter (700) angeordnet ist, der das Funktionsmaterial enthält;
einen Erfassungsteil (720), der vom Behälter Informationen erfasst, die eine Art des Funktionsmaterials im Behälter betreffen; und
einen Anzeigeteil (250), der Informationen anzeigt, die auf den durch den Erfassungsteil erfassten Informationen beruhen,
wobei die Ausstoßvorrichtung(500) aufweist:
ein Gehäuse (600), das am beweglichen Bedienungsfeld (200) befestigt ist;
einen Aufnahmeteil (510), der beweglich im Gehäuse (600) angeordnet ist;
einen Motor (630), der Leistung zum Bewegen des Aufnahmeteils (510) erzeugt; und
einen Leistungsübertragungsteil zum Übertragen der Leistung des Motors (630) auf den Aufnahmeteil (510),
wobei der Aufnahmeteil (510) aufweist:
eine Kammer (512) zum Aufnehmen des Behälters (700); und
einen Kanal (514), der mit einem Luftkanal im Außengehäuse (100) in Verbindung steht, und
wobei das aus dem Behälter (700) ausgestoßene Funktionsmaterial durch den Kanal (514) geht und aus den Ausstoßteilen (110,120) zusammen mit der Luft im Innenraum nach außen ausgestoßen wird.

2. Klimaanlage nach Anspruch 1, wobei die Ausstoßvorrichtung (500) ferner einen Ausstoßmechanismus (730) aufweist, der konfiguriert ist, das Funktionsmaterial aus dem Behälter auszustoßen.

3. Klimaanlage nach Anspruch 2, wobei der Aufnahmeteil (510) in das Außengehäuse (100) einführbar oder aus ihm herausziehbar ist.

4. Klimaanlage nach Anspruch 2 oder 3, wobei ein Abschnitt des Ausstoßmechanismus (730) in der Aufnahmekammer angeordnet ist, wenn sich der Aufnahmeteil (510) in seinem eingeführten Zustand befindet.

5. Klimaanlage nach Anspruch 4, wobei ein Abschnitt des Ausstoßmechanismus (730) zur Aufnahmekammer (512) vorsteht, wenn sich der Aufnahmeteil (510) in seinem herausgezogenen Zustand befindet.

6. Klimaanlage nach Anspruch 4, wobei der Ausstoßmechanismus (730) im Gehäuse angeordnet ist.

7. Klimaanlage nach einem der Ansprüche 2 bis 6, wobei der Ausstoßmechanismus (730) den Behälter (700) berührt, wenn er in den Aufnahmeteil (510) aufgenommen ist.

8. Klimaanlage nach einem der Ansprüche 2 bis 7, wobei der Ausstoßmechanismus (730) mindestens eine Elektrode (651, 652) zur Kontaktaufnahme mit dem Behälter (700) aufweist.

9. Klimaanlage nach Anspruch 7 oder 8, wobei der Ausstoßmechanismus (730) in den Behälter (700) eingeführt ist, während der Behälter (700) in den Aufnahmeteil (510) aufgenommen ist.

10. Klimaanlage nach Anspruch 7, 8 oder 9, wobei der Ausstoßmechanismus (730) konfiguriert ist, in den Behälter (700) eingeführt zu werden, während der Aufnahmeteil (510), in den der Behälter (700) aufgenommen ist, in das Außengehäuse (100) eingeführt wird oder nachdem der Aufnahmeteil (510), in den der Behälter (700) aufgenommen ist, in das Außengehäuse (100) eingeführt ist.

11. Klimaanlage nach einem der Ansprüche 2 bis 10, wobei der Ausstoßmechanismus (730) konfiguriert ist, einen Druck im Behälter (700) zu erfassen, während der Ausstoßmechanismus (730) in den Behälter (700) eingeführt wird, und die Art des Funktionsmaterials auf der Grundlage des erfassten Drucks zu erfassen.

12. Klimaanlage nach einem der Ansprüche 1 bis 11, wobei der Erfassungsteil (720) konfiguriert ist, die Montage des Behälters (700) und/oder die Art des Funktionsmaterials im Behälter (700) vorzugsweise durch ein Signal zu erfassen, das durch einen Eingabeteil eingegeben wird.

13. Klimaanlage nach einem der Ansprüche 1 bis 12, die ferner einen Steuerteil (740) aufweist, der konfiguriert ist, den Anzeigeteil (250) zum Anzeigen von Informationen zu steuern, um den Benutzer zu informieren, dass der Behälter (700) aufgenommen ist, wenn die Aufnahme des Behälters in die Ausstoßvorrichtung erfasst wird.

14. Klimaanlage nach einem der Ansprüche 1 bis 13, die ferner einen Steuerteil (740) aufweist, der konfiguriert ist, den Anzeigeteil (250) zu steuern, Informationen, die die Art des Funktionsmaterials betreffen, und/oder Wirkungsinformationen des Funktionsmaterials und/oder Ausstoßzeit-Informationen des Funktionsmaterials und/oder Betriebsinformationen anzuzeigen, um den Benutzer zu informieren, dass das Funktionsmaterial ausgestoßen wird.

15. Klimaanlage nach Anspruch 14, wobei der Steuerteil (740) konfiguriert ist, den Anzeigeteil (250) zu steuern, gleichzeitig oder aufeinanderfolgend mindestens zwei der Artinformationen, der Wirkungsinformationen, der Ausstoßzeit-Informationen und der Betriebsinformationen anzuzeigen.

## Revendications

1. Climatiseur, comprenant un boîtier (100) définissant un espace intérieur où de l'air circule pendant le fonctionnement du climatiseur ; une première et une deuxième sections de refoulement (110, 120) disposées de manière à être espacées l'une de l'autre sur le devant du boîtier (100), et par lesquelles de l'air est évacué ;
un panneau de commande mobile (200) disposé sur le devant du boîtier (100) entre la première et la deuxième sections de refoulement et couvrant au moins une partie de chaque section de refoulement (110, 120) ;
des panneaux de refoulement mobiles (310, 320) disposées sur au moins un côté du panneau de commande mobile (200) ;
un dispositif d'évacuation (500) disposé à l'intérieur du boîtier pour évacuer un matériau fonctionnel d'un réservoir (700) contenant ledit matériau fonctionnel ;
une section de détection (720) détectant du réservoir des informations relatives à un type de matériau fonctionnel dans le réservoir ; et
une section d'affichage (250) affichant des informations sur la base des informations détectées par la section de détection,
où le dispositif d'évacuation (500) comprend :
un boîtier (600) fixé au panneau de commande mobile (200) ;
une section de réception (510) disposée de manière mobile à l'intérieur du boîtier (600) ;
un moteur (630) générant une puissance pour déplacer la section de réception (510) ; et
une section de transmission de puissance destinée à transmettre la puissance du moteur (630) à la section de réception (510),
où la section de réception (510) comprend :
une chambre (512) destinée à recevoir le réservoir (700) ; et
un passage (514) communiquant avec un passage d'air à l'intérieur du boîtier (100), et où le matériau fonctionnel évacué du réservoir (700) passe dans le passage (514) et est évacué à l'extérieur par les sections de refoulement (110,120) avec l'air de l'espace intérieur.

2. Climatiseur selon la revendication 1, où le dispositif d'évacuation (500) comprend en outre un mécanisme d'évacuation (730) prévu pour évacuer le matériau fonctionnel du réservoir.

3. Climatiseur selon la revendication 2, où la section de réception (510) peut être introduite dans le boîtier (100) ou être retirée de celui-ci.

4. Climatiseur selon la revendication 2 ou la revendication 3,
où une partie du mécanisme d'évacuation (730) est disposée dans la chambre de réception quand la section de réception (510) est en état d'insertion.

5. Climatiseur selon la revendication 4, où une partie du mécanisme d'évacuation (730) fait saillie vers la chambre de réception (512) quand la section de réception (510) est en état de retrait.

6. Climatiseur selon la revendication 4, où le mécanisme d'évacuation (730) est disposé dans le boîtier.

7. Climatiseur selon l'une des revendications 2 à 6, où le mécanisme d'évacuation (730) contacte le réservoir (700) lorsqu'il est logé dans la section de réception (510).

8. Climatiseur selon l'une des revendications 2 à 7, où le mécanisme d'évacuation (730) comprend au moins une électrode (651, 652) pour contacter le réservoir (700).

9. Climatiseur selon la revendication 7 ou la revendication 8, où le mécanisme d'évacuation (730) est inséré dans le réservoir (700) alors que le réservoir (700) est logé dans la section de réception (510).

10. Climatiseur selon la revendication 7, la revendication 8 ou la revendication 9, où le mécanisme d'évacuation (730) est prévu pour être inséré dans le réservoir (700) quand la section de réception (510) où le réservoir (700) est logé est insérée dans le boîtier (100) ou après que la section de réception (510) où le réservoir est logé est insérée dans le boîtier (100).

11. Climatiseur selon l'une des revendications 2 à 10, où le mécanisme d'évacuation (730) est prévu pour détecter une pression à l'intérieur du réservoir (700) alors que le mécanisme d'évacuation (730) est inséré dans le réservoir (700), et pour détecter le type de matériau fonctionnel sur la base de la pression détectée.

12. Climatiseur selon l'une des revendications 1 à 11, où la section de détection (720) est prévue pour détecter le montage du réservoir (700) et/ou le type de matériau fonctionnel à l'intérieur du réservoir (700), préférentiellement par un signal appliqué par une section d'entrée.

13. Climatiseur selon l'une des revendications 1 à 12, comprenant en outre une section de commande (740) prévue pour commander l'affichage par la section d'affichage (250) d'informations avertissant l'utilisateur que le réservoir (700) est logé quand la réception du réservoir est détectée dans le dispositif d'évacuation.

14. Climatiseur selon l'une des revendications 1 à 13, comprenant en outre une section de commande (740) prévue pour commander l'affichage par la section d'affichage (250) d'informations relatives au type de matériau fonctionnel et/ou d'informations sur les effets du matériau fonctionnel et/ou d'informations de temps d'évacuation du matériau fonctionnel et/ou d'informations de fonctionnement avertissant que le matériau fonctionnel est évacué.

15. Climatiseur selon la revendication 14, où la section de commande (740) est prévue pour commander l'affichage simultané ou successif par la section d'affichage (250) d'au moins deux informations entre les informations relatives, les informations sur les effets, les informations de temps d'évacuation, et les informations de fonctionnement.
